# EUROPÄISCHE PATENTSCHRIFT

(11) **EP 0 708 154 B1**
(45) Veröffentlichungstag und Bekanntmachung des Hinweises auf die Patenterteilung: **15.03.2000**
(21) Anmeldenummer: 95116069.6
(22) Anmeldetag: 12.10.1995
(51) Int. Cl.: C09C 1/62, C09D 7/12, C08K 9/02, C03C 4/02, C04B 33/14, A61K 7/00, C09C 1/00

(54) **Mehrfach beschichtete metallische Glanzpigmente**
Brilliant metallic pigments bearing several coatings
Pigments métalliques brillants à plusieurs revêtements

(30) Priorität: 21.10.1994 DE 4437753
(43) Veröffentlichungstag der Anmeldung: 24.04.1996
(73) Patentinhaber: BASF AKTIENGESELLSCHAFT, 67056 Ludwigshafen (DE)
(72) Erfinder: Schmid, Raimund, Dr., D-67435 Neustadt (DE); Mronga, Norbert, Dr., D-69221 Dossenheim (DE)

(56) Entgegenhaltungen:
- EP-A- 0 338 428
- EP-A- 0 571 836
- EP-A- 0 579 091
- EP-A- 0 655 486
- DE-A- 4 313 541
- DE-A- 4 319 669

## Beschreibung

Die vorliegende Erfindung betrifft neue Glanzpigmente auf der Basis von mehrfach beschichteten plättchenförmigen metallischen Substraten, die mindestens ein Schichtpaket aus
A) einer farblosen Beschichtung mit einem Brechungsindex n ≤ 1,8 und
B) einer selektiv absorbierenden Beschichtung mit einem Brechungsindex n ≥ 2,0, die im wesentlichen aus den selektiv absorbierenden Oxiden Eisen(III)oxid, Chrom(III)oxid, Vanadium(V)oxid und/oder Titan(III)oxid oder aus den farblosen Oxiden Titandioxid und/oder Zirkonoxid besteht, die mit Hilfe von selektiv absorbierenden Farbmitteln eingefärbt worden sind,
sowie gewünschtenfalls zusätzlich
C) eine äußere, farblose oder selektiv absorbierende, von der darunterliegenden Schicht (B) verschiedene Beschichtung
aufweisen.

Weiterhin betrifft die Erfindung die Herstellung dieser Pigmente und ihre Verwendung zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Glanzpigmente auf Basis metallischer Substrate sind aufgrund ihres guten Deckvermögens auch für Automobillackierungen von besonderem Interesse.

Bislang sind folgende metallische Glanzpigmente bekannt:

In der EP-A-33 457 sind eisenoxidbeschichtete Aluminiumpigmente beschrieben, die im Glanzwinkel interessante goldene bis rote Farbtöne aufweisen. Diese Pigmente zeigen jedoch ebenso wie die aus der EP-A-338 428 oder der US-A-5 261 955 bekannten Aluminiumpigmente, die mit hochbrechenden Metalloxiden, nämlich mit reduziertem, blauem Titanoxid oder mit farblosem Titandioxid und Chrom- oder Eisenoxid bzw. mit einer Eisen(III)- oder Cobalt(II)-ionen enthaltenden Zirkonoxidschicht belegt sind, bei steileren Betrachtungswinkeln einen Wechsel von der jeweiligen, meist intensiven Absorptionsfarbe nach Unbunt. In der DE-A-43 19 669 sind mit Titandioxid und Molybdän(IV-VI)-Mischoxiden beschichtete Aluminiumpigmente beschrieben, bei denen ebenfalls die (blaue) Absorptionsfarbe vorherrscht.

Außerdem werden ausschließlich mit farblosen, hochbrechenden Metalloxiden wie Titandioxid oder Zirkondioxid oder auch mehrfach mit niedrigbrechenden Silicium- und hochbrechendem Titandioxid beschichtete Aluminiumpigmente beschrieben, die neben starkem metallischen Glanz nur zarte Interferenzfarben aufweisen (EP-A-338 428, US-A-5 213 618, JP-A-93 206/1994).

Besonders interessante, goniochromatische (sog. two-tone) Pigmente werden erhalten, wenn, wie in der EP-A-571 836 und der nicht vorveröffentlichten DE-A-44 05 492 beschrieben, Metalloxidbeschichtungen (insbesondere hochbrechendes Titandioxid bzw. niedrigbrechendes Siliciumdioxid) mit Metallbeschichtungen kombiniert werden. Glanzpigmente mit ähnlichen Schichtenabfolgen sind aus den US-A-5 135 812 und 3 438 796 bekannt. Aufgrund ihrer Herstellungsweise ist bei diesen Pigmenten der zentrale Metallfilm nicht vollständig an allen Seiten von den äußeren Schichten umhüllt.

Diese koloristisch interessanten Pigmente können jedoch aufgrund der äußeren Metallbeschichtungen insbesondere in Automobillacken zu Problemen hinsichtlich ihrer Echtheit führen. Außerdem lassen sich mit diesen Pigmenten nicht alle gewünschten Farbflops erreichen.

Der Erfindung lag die Aufgabe zugrunde, farbstarke Glanzpigmente bereitzustellen, die eine attraktive Koloristik und vorteilhafte Anwendungseigenschaften aufweisen.

Demgemäß wurden die eingangs definierten Glanzpigmente gefunden.

Weiterhin wurde ein Verfahren zur Herstellung der Glanzpigmente gefunden, welches dadurch gekennzeichnet ist, daß man die metallischen Substratteilchen durch Gasphasenzersetzung flüchtiger Metallverbindungen in Gegenwart von Sauerstoff und/oder Wasserdampf oder naßchemisch durch hydrolytische Zersetzung organischer Metallverbindungen nacheinander mit den einzelnen Schichten belegt.

Schließlich wurde die Verwendung der Glanzpigmente zur Einfärbung von Lacken, Druckfarben, Tinten, Kunststoffen, Gläsern, keramischen Produkten und Zubereitungen der dekorativen Kosmetik gefunden.

Die erfindungsgemäßen metallischen Glanzpigmente weisen eine farblose, niedrigbrechende Beschichtung (A) in Kombination mit einer selektiv absorbierenden (d.h. farbigen, aber nicht schwarzen), hochbrechenden, im wesentlichen aus den eingangs definierten Metalloxiden bestehenden Beschichtung (B) sowie gewünschtenfalls eine zusätzliche äußere, farblose oder selektiv absorbierende Beschichtung (C), die niedrig- oder hochbrechend sein kann und von der darunterliegenden Schicht (B) verschieden ist, auf und können mehrere, gleiche oder verschiedene Kombinationen (A) + (B) enthalten; bevorzugt ist jedoch die Belegung mit nur einem Schichtpaket (A) + (B).

Die Beschichtung (A) hat in der Regel einen Brechungsindex n ≤ 1,8, bevorzugt ≤ 1,6, während die Beschichtung (B) im allgemeinen einen Brechungsindex n ≥ 2,0, vorzugsweise ≥ 2,4 aufweist.

Besonders bevorzugte Kombinationen (A) + (B) zeigen eine Differenz der Brechungsindizes von in der Regel ≥ 0,4, insbesondere ≥ 0,8.

Als Materialien eignen sich für die Beschichtung (A) alle Substanzen, die filmartig und dauerhaft auf die Substratteilchen aufgebracht werden können und die erforderlichen optischen Eigenschaften haben. Besonders geeignet sind natürlich (vor allem für die Beschichtung (B)) solche Materialien, die den Echtheitsanforderungen bei der Anwendung genügen.

Bevorzugte Beispiele für die Schichtmaterialien (A) sind Metalloxide, die farblos und niedrigbrechend sind.

Im einzelnen seien für die Beschichtung (A) beispielhaft genannt: Siliciumoxid, Siliciumoxidhydrat, Aluminiumoxid, Aluminiumoxidhydrat und deren Mischungen, wobei Siliciumoxid(hydrat) bevorzugt ist.

Weitere für die Beschichtung (A) geeignete Materialien sind z.B. Magnesiumfluorid und Aluminiumphosphat.

Die Beschichtung (B) besteht im wesentlichen aus den eingangs definierten hochbrechenden Metalloxiden, die vorzugsweise "von selbst" selektiv absorbierend sind (insbesondere Eisen(III)oxid (α- und γ-Fe₂O₃, rot), auch Chrom(III)oxid (grün), Titan(III)oxid (blau; liegt aufgrund der üblichen Herstellung durch Reduktion von TiO₂ mit Ammoniak in der Regel im Gemisch mit Titanoxynitriden und Titannitriden vor) und Vanadiumpentoxid (orange) sowie deren Mischungen) oder farblos sind (Titandioxid und/oder Zirkonoxid) und mit selektiv absorbierenden Farbmitteln "eingefärbt" werden.

Die "Einfärbung" kann hierbei durch den Einbau der Farbmittel in die Metalloxidschicht bzw. durch deren Dotierung mit selektiv absorbierenden Metallkationen oder durch Überziehen der Metalloxidschicht mit einem das Farbmittel enthaltenden Film erfolgen.

Als Farbmittel sind z.B. anorganische und organische Pigmente, Küpenfarbstoffe sowie weitere organische Farbstoffe, die sich in einen stabilen Polymerfilm einbauen lassen, geeignet.

Gewünschtenfalls kann die "eingefärbte" Beschichtung (B) noch durch eine Schicht (C) stabilisiert werden.

Im einzelnen seien z.B. folgende Farbmittel genannt: Eisenoxide, Bismutvanadat, farbige Spinelle, Nickeltitangelb und Cyanokomplexe des Eisens (Berliner Blau); Monoazopigmente (z.B. Produkte, die sich von Acetessigarylidderivaten oder von β-Naphtholderivaten ableiten), verlackte Monoazofarbstoffe, wie verlackte β-Hydroxynaphthoesäurefarbstoffe, Disazopigmente, kondensierte Disazopigmente, Isoindolinderivate, Derivate der Naphthalin- oder Perylentetracarbonsäure, Anthrachinonpigmente, Thioindigoderivate, Azomethinderivate, Chinacridone, Dioxazine, Diketopyrrolopyrrole, Pyrazolochinazolone, Phthalocyaninpigmente und verlackte basische Farbstoffe, wie verlackte Triarylmethanfarbstoffe.

Der Einbau von Pigmenten in die Metalloxidschicht (B) kann in einfacher Weise erreicht werden, indem man ein anorganisches Salz oder Alkoholat des Metalls in wäßriger oder z.B. alkoholischer Lösung hydrolysiert und gemeinsam mit dem Pigment auf die Substratteilchen auffällt.

Die Dotierung der Metalloxidschicht (B) mit farbigen Metallkationen kann in ähnlicher Weise durch Mitreißen bei der Oxidausfällung erfolgen.

Ein geeigneter farbmittelhaltiger Überzug kann z.B. ein farbiger Polymerfilm, der durch Copolymerisation der Monomeren in Gegenwart von gelöstem Farbstoff aufgebracht wird oder an den ein Pigment adsorbiert wird, oder ein Küpenfarbstoffilm sein, der durch Oxidation der gelösten Leukoform auf die Substratteilchen aufgefällt wird.

Die genannten Methoden sind in der Patentliteratur vielfach beschrieben und können daher vom Fachmann ohne weiteres durchgeführt werden.

Die erfindungsgemäßen Glanzpigmente können noch eine zusätzliche Beschichtung (C) als Deckschicht aufweisen, die z.B. zum Schutz einer Ti₂O₃-Schicht (B) oder zur Verbesserung der Dispergierbarkeit dienen kann.

Für die Beschichtung (C) eignen sich farblose oder selektiv absorbierende Metalloxide, je nachdem, ob die Koloristik (Farbe) des Pigments zusätzlich modifiziert werden soll oder nicht. Die Oxide können sowohl niedrigbrechend als auch hochbrechend sein. Als Beispiele seien Siliciumoxid, Siliciumoxidhydrat, Aluminiumoxid, Aluminiumoxidhydrat, Zinnoxid, Titandioxid, Zirkonoxid, Eisen(III)oxid und Chrom(III)oxid genannt. Bevorzugt ist Siliciumoxid(hydrat).

Bei den erfindungsgemäßen Glanzpigmenten haben die einzelnen Beschichtungen im allgemeinen folgende Schichtdicken:
(A) 10 bis 800 nm, vorzugsweise 50 bis 600 nm;
(B) 1 bis 500 nm, vorzugsweise 10 bis 150 nm;
(C) 1 bis 200 nm, vorzugsweise 10 bis 150 nm.

Sind mehrere (z.B. 2, 3 oder 4) Schichtpakete (A) + (B) enthalten, dann ist die Beschichtung (A) bevorzugt 10 bis 200 nm dick und die Beschichtung (B) vorzugsweise 10 bis 50 nm dick.

Als metallische Substrate kommen bei den erfindungsgemäßen Glanzpigmenten alle für Metalleffektpigmente bekannten Metalle und Legierungen in Plättchenform in Betracht; z.B. sind neben Stahl, Kupfer und seinen Legierungen wie Messing und Bronzen vor allem Aluminium und seine Legierungen wie Aluminiumbronze geeignet.

Bevorzugt sind Aluminiumflakes, die in einfacher Weise durch Herausstanzen aus Aluminiumfolie oder nach gängigen Verdüsungs- und Mahltechniken herzustellen sind.

So sind beispielsweise Aluminiumpigmente geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschriebenen, durch oxidative Behandlung entfernt werden.

Weiterhin können die metallischen Substratteilchen passiviert sein, d.h. insbesondere gegenüber Wasser stabilisierende Beschichtungen aufweisen, wie sie z.B. aus den nicht vorveröffentlichten DE-A-42 36 332 und 44 14 079 bekannt sind.

Die metallischen Substratteilchen können gewünschtenfalls auch mit Metalloxid wie Eisen- oder Titanoxid beschichtet sein und daher durch Interferenzeffekte und gegebenenfalls Absorption bereits eine (schwache) Eigenfarbe besitzen. Jedoch sollte die Metalloxidschicht nicht zu dick sein, damit die Substratteilchen ihre "metallische Koloristik" behalten.

Schließlich sind auch magnetisierbare Aluminiumplättchen geeignet, die eine Eisen-, Cobalt-, Nickel- oder γ-Fe₂O₃-Beschichtung aufweisen (nicht vorveröffentlichte DE-A-43 13 541 und 43 40 141).

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von etwa 0,1 bis 5 µm, insbesondere um etwa 0,5 µm. Ihre spezifische freie Oberfläche (BET) beträgt im allgemeinen 0,1 bis 5 m²/g.

Die erfindungsgemäßen Glanzpigmente haben neben insgesamt vorteilhaften Anwendungseigenschaften (insbesondere auch guten Echtheiten) vor allem auch interessante koloristische Eigenschaften. Insbesondere zeigen sie Farbflops, die mit analogen eine metallische Schicht (B) aufweisenden Glanzpigmenten nicht zu erhalten sind.

Beispielsweise wird bei einem Pigment mit der Schichtfolge Aluminium/SiO₂/Molybdän, das in Aufsicht eine rotgoldene Interferenzfarbe zeigt, bei steileren Betrachtungswinkeln eine grüngoldene Interferenzfarbe sichtbar. Ein vergleichbares, anstelle von Molybdän mit Fe₂O₃ beschichtetes Pigment weist dagegen einen Farbflop von rotgold nach intensiv rot auf.

So werden bei Beschichtung mit Eisenoxid (B) Glanzpigmente für den roten Farbtonbereich erhalten, die bei einer dünnen Eisenoxidschicht einen Farbwechsel von rotgold → grüngold zeigen, der sich mit steigender Fe₂O₃-Schichtdicke verändert auf orangerot → intensiv rot. Man kann also durch Variation der Schichtdicke der SiO₂- und/oder der Fe₂O₃-Schicht eine ganze Palette von Rottönen einstellen, die jeweils bei wechselnden Betrachtungswinkeln nach grüngold, gold oder rotgold abkippen.

Bei Beschichtung mit Chromoxid (B) ergeben sich entsprechend Glanzpigmente mit einer Vielzahl von Grüntönen, die Farbwechsel nach blau oder grüngold aber auch rot zeigen.

Blaue Beschichtungen (B) (Ti₂O₃) liefern Glanzpigmente mit Farbflops im violetten bzw. blauen Farbtonbereich.

Bei dem erfindungsgemäßen Verfahren zur Herstellung der Glanzpigmente werden die einzelnen Schichten durch Gasphasenzersetzung geeigneter flüchtiger Metallverbindungen (chemical vapor deposition, CVD) oder naßchemisch durch hydrolytische Zersetzung insbesondere organischer Metallverbindungen aufgebracht.

Selbstverständlich können beide Vorgehensweisen beliebig zur Herstellung der einzelnen Schichten kombiniert werden.

Zur Erzeugung der Metalloxidschichten (A) sind die naßchemische und die CVD-Verfahrensvariante gleichermaßen geeignet, jedoch wird meist die CVD-Variante vorzuziehen sein, da sich die bevorzugten Metalloxidschichten (B) besonders vorteilhaft ebenfalls aus der Gasphase abscheiden lassen. Eine Zwischenisolierung und Trocknung des mit (A) belegten Pigments kann dann entfallen.

Bei der in der nicht vorveröffentlichten DE-A-44 05 492 beschriebenen naßchemischen Variante werden organische Silicium- und/oder Aluminiumverbindungen, bei denen die organischen Reste über Sauerstoffatome an die Metalle gebunden sind, in Gegenwart der Substratteilchen und eines organischen Lösungsmittels, in welchem die Metallverbindungen löslich sind, hydrolysiert.

Hierfür sind eine Vielzahl von organischen Lösungsmitteln geeignet, bevorzugt ist Isopropanol.

Bevorzugte Beispiele für die metallischen Ausgangsverbindungen sind die Acetylacetonate und insbesondere Alkoholate, vor allem C₁-C₄-Alkanolate, z.B. Aluminiumtriisopropanolat und Tetraethoxysilan.

Die Hydrolyse wird vorzugsweise in Gegenwart einer Base oder einer Säure als Katalysator durchgeführt. Hierfür eignen sich z.B. neben Alkalilaugen wie Natronlauge insbesondere wäßrige Ammoniaklösungen. Geeignete saure Katalysatoren sind beispielsweise Phosphorsäure und organische Säuren wie Essigsäure und Oxalsäure.

Wasser muß mindestens in der stöchiometrisch für die Hydrolyse erforderlichen Menge vorliegen, bevorzugt ist jedoch die 2 bis 100fache, insbesondere die 5 bis 20fache Menge.

Bezogen auf die eingesetzte Wassermenge, gibt man in der Regel 3 bis 40 Vol.-%, vorzugsweise 5 bis 30 Vol.-%, einer 25 gew.-%igen wäßrigen Ammoniaklösung zu.

Für die Temperaturführung hat es sich als vorteilhaft erwiesen, das Reaktionsgemisch innerhalb von 10 bis 48 h schrittweise auf Rückflußtemperatur zu erhitzen. Bei Verwendung von iso-Propanol als Lösungsmittel rührt man das Gemisch zum Beispiel bevorzugt zunächst 4 bis 20 h bei 40°C, dann 4 bis 20 h bei 60°C und zum Schluß 2 bis 8 h bei 80°C.

Verfahrenstechnisch geht man bei Schritt a) des erfindungsgemäßen Herstellungsverfahrens zweckmäßigerweise wie folgt vor:
Man legt Substratteilchen, organisches Lösungsmittel, Wasser und Katalysator (Säure oder bevorzugt Base, insbesondere z.B. eine wäßrige Ammoniaklösung) vor und gibt die zu hydrolysierende Metallverbindung, pur oder gelöst, z.B. als 30 bis 70, bevorzugt 40 bis 60 vol.-%ige Lösung im organischen Lösungsmittel, zu. Erfolgt die Zugabe der Metallverbindung in einem Schritt, dann wird die Suspension anschließend wie oben beschrieben unter Rühren erhitzt. Man kann die Metallverbindung aber auch bei erhöhter Temperatur kontinuierlich zudosieren, wobei Wasser und Ammoniak vorgelegt oder ebenfalls kontinuierlich zudosiert werden können. Nach beendeter Beschichtung wird die Reaktionsmischung wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gewünschtenfalls kann man den Beschichtungsschritt ein- oder mehrfach wiederholen. Sollte die Mutterlauge milchig trüb aussehen, so empfiehlt es sich, diese vor einer weiteren Beschichtung auszutauschen.

Die Isolierung der mit der Schicht (A) belegten Substratteilchen kann in einfacher Weise durch Abfiltrieren, Waschen mit organischem Lösungsmittel, vorzugsweise den auch als Lösungsmittel verwendeten Alkoholen, und anschließendes Trocknen (üblicherweise 2 bis 24 h bei 20 bis 200°C) erfolgen.

Bei der in der ebenfalls anhängigen deutschen Patentanmeldung P 44 37 752.5 beschriebenen CVD-Variante werden Silane, die mindestens einen Alkanoylrest enthalten, in der Gasphase mit Wasserdampf und gegebenenfalls Sauerstoff in Gegenwart bewegter Substratteilchen zersetzt.

Hierfür geeignete Silane entsprechen insbesondere der Formel

RₐSiX_{b}Y_{c}

in der die Variablen folgende Bedeutung haben:
- R: Alkyl, bevorzugt C₁-C₁₀-Alkyl, besonders bevorzugt C₁-C₆-Alkyl, das durch Chlor substituiert sein kann, ein- oder mehrfach ungesättigt sein kann und dessen Kohlenstoffkette durch eine oder mehrere Iminogruppen oder Sauerstoffatome in Etherfunktion unterbrochen sein kann; Phenyl, das durch C₁-C₂-Alkyl substituiert sein kann, oder Wasserstoff;
- X: Alkoxy, bevorzugt C₁-C₆-Alkoxy, besonders bevorzugt C₄-Alkoxy, vor allem tert.-Butoxy;
- Y: Alkanoyloxy, bevorzugt C₂-C₃-Alkanoyloxy, besonders bevorzugt Acetoxy;
- a: 0 bis 3, bevorzugt 0 bis 2, besonders bevorzugt 0;
- b: 0 bis 3, bevorzugt 1 bis 3, besonders bevorzugt 2;
- c: 1 bis 4, bevorzugt 1 bis 3, besonders bevorzugt 2,
wobei die Summe a+b+c=4 ist und die Reste R für a>1, die Reste X für b>1 und die Reste Y für c>1 jeweils gleich oder verschieden sein können.

Besonders geeignet sind die Silane, die bei Temperaturen ≤ 600°C, aus technischen Gründen insbesondere auch ≤ 300°C, einen ausreichend hohen Dampfdruck aufweisen, um eine einfache Verdampfung zu gewährleisten, und auch leicht mit Wasserdampf und/oder Luft zersetzt und als Oxid abgeschieden werden können. Selbstverständlich können auch Gemische verschiedener Silane eingesetzt werden.

Im einzelnen seien beispielhaft folgende bevorzugte Silane genannt:

Tetraacetoxysilan, Methoxy-, Ethoxy-, Propoxy-, Isopropoxy-, Butoxy-, Isobutoxy-, sec.-Butoxy- und tert.-Butoxytriacetoxysilan, Dimethoxy-, Diethoxy-, Dipropoxy-, Diisopropoxy-, Dibutoxy-, Diisobutoxy-, Di-sec.-butoxy- und Di-tert.-butoxydiacetoxysilan und Trimethoxy-, Triethoxy-, Tripropoxy-, Triisopropoxy-, Tributoxy-, Triisobutoxy-, Tri-sec.-butoxy- und Tri-tert.-butoxyacetoxysilan sowie auch Silane, die verschiedene Alkoxyreste enthalten, z.B. Methoxyethoxydiacetoxysilan.

Ganz besonders bevorzugt ist Di-tert.-butoxydiacetoxysilan.

Zur Durchführung der CVD-Variante empfiehlt sich wie allgemein für CVD-Verfahren die Verwendung eines Wirbelschichtreaktors, wie er beispielsweise in der EP-A 45 851 beschrieben ist. Die Substratteilchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (sowie gegebenenfalls Sauerstoff) werden dann mit Hilfe inerter Trägergasströme (vorteilhaft Teilströmen des Wirbelgases) aus vorgeschalteten Verdampfergefäßen über getrennte Düsen eingeleitet.

Um homogene, die Substratteilchen vollständig umhüllende, filmartige Siliciumoxidschichten zu erhalten, wird die Silankonzentration zweckmäßigerweise bei ≤ 5 Vol.-%, vorzugsweise ≤ 2 Vol.-%, bezogen auf die Gesamtgasmenge im Reaktor, gehalten.

Die zur Zersetzung erforderliche Menge Wasserdampf hängt von der Konzentration des Silans ab und sollte mindestens der stöchiometrisch zur Hydrolyse erforderlichen Menge entsprechen, bevorzugt ist jedoch die 10 bis 100fache Menge.

Enthält das Silan Alkyl- oder Phenylsubstituenten R, so empfiehlt sich die Anwesenheit von Sauerstoff bei der Zersetzung, wenn Kohlenstoffreste, die sich in der Regel bei der alleinigen Verwendung von Wasserdampf bilden, in der abgeschiedenen Siliciumoxidschicht vermieden werden sollen.

Zum Aufbringen der Metalloxidschichten (B) eignet sich aufgrund der hohen Qualität der abgeschiedenen Schichten insbesondere das CVD-Verfahren, sollen jedoch "eingefärbte" Titan- oder Zirkondioxidschichten als Beschichtung (B) dienen, kann je nach der "Einfärbungsmethode" der naßchemische Verfahrensweg zweckmäßiger sein (z.B. EP-A-328 906).

Die CVD-Varianten zur Abscheidung von α-Eisen(III)oxid, Chrom(III)oxid, Titandioxid und Titan(III)oxid (im Gemisch mit Titanoxynitriden und Titannitriden) durch oxidative Zersetzung von Eisenpentacarbonyl und Chromhexacarbonyl bzw. hydrolytische Zersetzung von Titantetraisopropanolat oder Titantetrachlorid sowie anschließende Reduktion mit Ammoniak sind hinlänglich bekannt (EP-A-33 457, EP-A-338 428).

Naßchemisch könnten α-Eisenoxid- und Chromoxidschichten durch hydrolytische Zersetzung von Eisen(III)salzen wie Eisen(III)chlorid und -sulfat bzw. Chrom(III)chlorid und anschließendes Überführen der gebildeten hydroxidhaltigen Schichten durch Tempern in die Oxidschichten aufgebracht werden. Ebenso könnte eine Titan(III)oxidbeschichtung durch Hydrolyse von Titantetrachlorid und anschließende Reduktion des gebildeten Titandioxids mit gasförmigem Ammoniak erreicht werden.

Die Belegung mit einer γ-Fe₂O₃-Schicht kann nach den beiden in der nicht vorveröffentlichten DE-A-43 40 141 beschriebenen CVD-Verfahrensvarianten erfolgen. Entweder kann Eisenpentacarbonyl in Gegenwart mindestens der stöchiometrisch erforderlichen Menge, besser mit der 10- bis 100-fachen Menge, Wasserdampf bei 180 bis 250°C zu Magnetit, Wasserstoff und Kohlenmonoxid zersetzt und der abgeschiedene Magnetitfilm anschließend bei 200 bis 350°C mit Luft zu γ-Fe₂O₃ oxidiert werden, oder Eisenpentacarbonyl kann zunächst durch oxidative Zersetzung als α-Fe₂O₃ abgeschieden werden, welches dann bei 200 bis 400°C mit wasserstoffhaltigen Gasen zu eisen(II)haltigen Produkten reduziert und anschließend wie oben zu γ-Fe₂O₃ oxidiert werden kann.

Vanadium(V)oxidschichten können schließlich durch Gasphasenzersetzung von Vanadiumoxychlorid mit Wasserdampf abgeschieden werden.

Falls eine äußere Metalloxidschicht (C) erwünscht ist, kann diese wie für die Schichten (A) und (B) beschrieben aufgebracht werden.

Mit Hilfe des erfindungsgemäßen Herstellungsverfahrens können die mehrfach beschichteten Glanzpigmente in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und insbesondere von Lacken, Tinten und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und dekkenden Weiß-, Bunt- und Schwarzpigmenten sowie auch herkömmlichen Glanzpigmenten auf der Basis von metalloxidbeschichteten Glimmer- und Metallpigmenten und plättchenförmigen Eisenoxiden verwenden.

### Beispiele

### Herstellung von erfindungsgemäßen Glanzpigmenten

### Beispiel 1

a) In einem mit Rückflußkühler und Rührapparatur versehenen Rundkolben wurden 200 g Aluminiumpulver (mittlerer Teilchendurchmesser 60 µm, BET-Oberfläche 1,5 m²/g) in 1,5 l Iso-propanol aufgeschlämmt. Nach Zugabe von 600 ml Wasser und 40 ml einer 25 gew.-%igen wäßrigen Ammoniaklösung wurde die Suspension unter kräftigem Rühren auf 60°C erhitzt. Dabei wurde gleichzeitig mit der Zudosierung eines Gemisches aus 610 ml Isopropanol und 610 g Tetraethoxysilan (Dosiergeschwindigkeit 130 ml/h) begonnen. Nach beendeter Zudosierung (etwa 10 h) wurde noch 14 h bei 55°C nachgerührt.
   Nach dem Abkühlen der Suspension wurde das Produkt von der Mutterlauge abfiltriert, mit Isopropanol gewaschen und bei 80°C getrocknet.
   Das beschichtete Aluminiumpulver hatte einen SiO₂-Gehalt von 42,7 Gew.-% und zeigte einen schwachen Grünstich.
b) Zur anschließenden Beschichtung mit α-Eisen(III)oxid wurden 160 g des getrockneten Produkts im Wirbelschichtreaktor (beschrieben in der EP-A-571 836) unter Fluidisierung mit insgesamt 900 l/h Stickstoff auf 200°C erhitzt. Aus einer auf Raumtemperatur gehaltenen Vorlage wurden mit einem Teil der Wirbelgase (300 l/h) 116 g Eisenpentacarbonyl in 8 h in den Reaktor überführt und dort unter gleichzeitiger Zuführung von 200 l/h Luft über eine weitere Düse zu α-Fe₂O₃ und Kohlenmonoxid bzw. Kohlendioxid zersetzt.

Das erhaltene Pigment hatte einen Eisengehalt von 12,4 Gew.-% und zeigte, im Lack appliziert, bei nahezu unverändert starkem metallischen Glanz eine kräftige blaustichig rote Interferenzfarbe, die bei steileren Betrachtungswinkeln ins Goldene abkippte.

Eine nach der Zufuhr von 87 g Fe(CO)₅ entnommene Pigmentprobe (Eisengehalt 9,2 Gew.-%) zeigte einen Farbflop von zartrot nach grünlich gold.

### Beispiel 2

a) Das gleiche Aluminiumpulver wurde analog zu Beispiel 1a) unter Verwendung eines Gemisches von 550 ml Isopropanol und 550 g Tetraethoxysilan mit SiO₂ beschichtet.
   Das beschichtete Aluminiumpulver hatte einen SiO₂-Gehalt von 39,6 Gew.-% und zeigte einen schwachen Blaustich.
b) Anschließend wurden 210 g des getrockneten Produkts analog zu Beispiel 1b), jedoch unter Fluidisierung mit insgesamt 1000 l/h Stickstoff bei 190°C unter Zufuhr von 175 g Fe(CO)₅ (Teilstrom 400 l/h) in 12 h mit α-Fe₂O₃ beschichtet.

Das erhaltene Pigment hatte einen Eisengehalt von 14,9 Gew.-% und zeigte, im Lack appliziert, bei starkem metallischem Glanz eine kräftige goldene Interferenzfarbe, die bei steileren Betrachtungswinkeln nach intensiv rot abkippte.

Eine nach der Zufuhr von 145 g Fe(CO)₅ entnommene Pigmentprobe (Eisengehalt 12,3 Gew.-%) zeigte einen Farbflop von rotgold nach gold.

### Beispiel 3

a) Das gleiche Aluminiumpulver wurde analog zu Beispiel 1a) unter Verwendung eines Gemisches von 690 ml Isopropanol und 690 g Tetraethoxysilan mit SiO₂ beschichtet.
   Das beschichtete Aluminiumpulver hatte einen SiO₂-Gehalt von 52,4 Gew.-% und zeigte einen rötlichen Schimmer.
b) Analog zu Beispiel 2b) wurden anschließend 200 g des getrockneten Produkts unter Verwendung von 87 g Fe(CO)₅ in 5 h mit α-Fe₂O₃ beschichtet.

Das erhaltene Pigment hatte einen Eisengehalt von 11,3 Gew.-% und zeigte, im Lack appliziert, bei starkem metallischen Glanz eine intensiv goldene Interferenzfarbe, die bei steileren Betrachtungswinkel nach schwach rot abkippte.

Eine nach der Zufuhr von 43,5 g Fe(CO)₅ entnommene Pigmentprobe (Eisengehalt 5,6 Gew.-%) zeigte einen Farbflop von schwach gold nach rot.

### Beispiel 4

a) In einem zu Beispiel 1 b) baugleichen, jedoch größer ausgelegten Wirbelschichtreaktor (Durchmesser 16 cm, Höhe 100 cm) wurden 500 g Aluminiumpulver (mittlerer Teilchendurchmesser 60 µm, spezifische Oberfläche 1,5 m²/g) unter Fluidisierung mit insgesamt 1420 l/h Stickstoff auf 200°C erhitzt. Ein Teil der Wirbelgase (400 l/h) wurde dabei über eine auf 50°C temperierte Wasservorlage geleitet. Zur Entfettung des Aluminiumpulvers wurden in 1 h über eine weitere Düse 140 l Luft zudosiert.
   Aus einer weiteren, auf 160°C erhitzten Verdampfervorlage wurden mit ebenfalls 400 l/h des Wirbelgases insgesamt 775 ml Di-tert.-butoxydiacetoxysilan in 25 ml-Portionen in 20,5 h in den Reaktor überführt und dort zu sich auf dem Aluminium abgeschiedenen SiO₂, tert.-Butanol und Essigsäure zersetzt.
   Eine Pigmentprobe hatte einen SiO₂-Gehalt von 25,0 Gew.-% und sah nahezu unverändert metallisch aus.
b) Zur anschließenden Belegung mit α-Fe₂O₃ wurde die Wirbelbetttemperatur auf 190°C eingestellt, die Silanvorlage wurde gegen eine auf Raumtemperatur gehaltene Vorlage mit Fe(CO)₅ ausgetauscht. Unter Verwendung von insgesamt 1600 l/h Stickstoff als Wirbelgas wurden so 130 g Fe(CO)₅ mit einem Teilstrom von 400 l/h Stickstoff in 8 h in den Reaktor überführt und dort mit 300 l/h Luft, die über die Wasservorlage eingeleitet wurde, oxidativ zersetzt.

Das erhaltene Pigment hatte einen Eisengehalt von 5,0 Gew.-% und zeigte, im Lack appliziert, bei nahezu unverändert starkem metallischen Glanz eine kräftige rotgoldene Interferenzfarbe, die bei steileren Betrachtungswinkeln nach grünlich gold abkippte.

### Beispiel 5

a) Das gleiche Aluminiumpulver wurde analog zu Beispiel la) unter Verwendung eines Gemisches von 730 ml Isopropanol und 730 g Tetraethoxysilan mit SiO₂ beschichtet.
   Das beschichtete Aluminiumpulver hatte einen SiO₂-Gehalt von 49,0 Gew.-% und zeigte einen grünlichen Schimmer.
b) Anschließend wurden 250 g des getrockneten Produkts in dem Wirbelschichtreaktor aus Beispiel 1b) unter Fluidisierung mit insgesamt 1000 l/h Stickstoff bei 220°C unter Zufuhr von 50,6 g Chromhexacarbonyl aus einer auf 70°C temperierten Vorlage (Wirbelgasteilstrom 400 l/h) und gleichzeitiger Einleitung von 200 l/h Luft in 20 h mit Chrom(III)oxid beschichtet.

Das erhaltene Pigment hatte einen Chromgehalt von 4,3 Gew.-% und zeigte, im Lack appliziert, bei starkem metallischen Glanz eine kräftige grüne Interferenzfarbe, die bei steileren Betrachtungswinkeln nach rot abkippte.

## Patentansprüche

1. Glanzpigmente auf der Basis von mehrfach beschichteten plättchenförmigen metallischen Substraten, die mindestens ein Schichtpaket aus
A) einer farblosen Beschichtung mit einem Brechungsindex n ≤ 1,8 und
B) einer selektiv absorbierenden Beschichtung mit einem Brechungsindex n ≥ 2,0, die im wesentlichen aus den selektiv absorbierenden Oxiden Eisen(III)oxid, Chrom(III)oxid, Vanadium(V)oxid und/oder Titan(III)oxid oder aus den farblosen Oxiden Titandioxid und/oder Zirkonoxid besteht, die mit Hilfe von selektiv absorbierenden Farbmitteln eingefärbt worden sind,
sowie gewünschtenfalls zusätzlich
C) eine äußere, farblose oder selektiv absorbierende, von der darunterliegenden Schicht (B) verschiedene Beschichtung
aufweisen.

2. Glanzpigmente nach Anspruch 1, bei denen die Beschichtung (A) eine im wesentlichen aus niedrigbrechendem, farblosem Metalloxid bestehende Schicht ist.

3. Glanzpigmente nach Anspruch 1 oder 2, bei denen die Beschichtung (A) im wesentlichen aus Siliciumoxid, Siliciumoxidhydrat, Aluminiumoxid und/oder Aluminiumoxidhydrat besteht.

4. Glanzpigmente nach den Ansprüchen 1 bis 3, bei denen die Beschichtung (C) im wesentlichen aus Siliciumoxid, Siliciumoxidhydrat, Aluminiumoxid, Aluminiumoxidhydrat, Titandioxid, Zirkonoxid, Eisen(III)oxid und/oder Chrom(III)oxid besteht.

5. Glanzpigmente nach den Ansprüchen 1 bis 4, die nur ein Schichtpaket aus (A) und (B) enthalten.

6. Glanzpigmente nach den Ansprüchen 1 bis 5, bei denen das metallische Substrat im wesentlichen aus Aluminiumplättchen, die passiviert sein können, besteht.

7. Verfahren zur Herstellung von Glanzpigmenten gemäß den Ansprüchen 1 bis 6, dadurch gekennzeichnet, daß man die metallischen Substratteilchen durch Gasphasenzersetzung flüchtiger Metallverbindungen in Gegenwart von Sauerstoff und/oder Wasserdampf oder naßchemisch durch hydrolytische Zersetzung organischer Metallverbindungen nacheinander mit den einzelnen Schichten belegt.

8. Verwendung von Glanzpigmenten gemäß den Ansprüchen 1 bis 6 zur Einfärbung von Lacken, Druckfarben, Tinten, Kunststoffen, Gläsern, keramischen Produkten und Zubereitungen der dekorativen Kosmetik.

## Claims

1. Luster pigments based on multiply coated plateletlike metallic substrates with at least one layer packet, comprising
A) a colorless coating having a refractive index n ≤ 1.8, and
B) a selectively absorbing coating having a refractive index n ≥ 2.0 which consists essentially of the selectively absorbing oxides iron(III) oxide, chromium(III) oxide, vanadium(V) oxide and/or titanium(III) oxide or of the colorless oxides titanium dioxide and/or zirconium oxide which have been colored with the aid of selectively absorbing colorants
and also if desired additionally
C) an outer, colorless or selectively absorbing coating different than the layer (B) underneath.

2. Luster pigments as claimed in claim 1 wherein coating (A) is a layer consisting essentially of a colorless metal oxide having a low refractive index.

3. Luster pigments as claimed in claim 1 or 2 wherein coating (A) consists essentially of silicon oxide, silicon oxide hydrate, aluminum oxide and/or aluminum oxide hydrate.

4. Luster pigments as claimed in any of claims 1 to 3 wherein coating (C) consists essentially of silicon oxide, silicon oxide hydrate, aluminum oxide, aluminum oxide hydrate, titanium dioxide, zirconium oxide, iron(III) oxide and/or chromium(III) oxide.

5. Luster pigments according to any of claims 1 to 4 wherein there is only one layer packet (A) and (B).

6. Luster pigments according to any of claims 1 to 5 wherein the metallic substrate consists essentially of aluminum platelets, passivated or unpassivated.

7. A process for producing luster pigments as claimed in any of claims 1 to 6, which comprises coating the metallic substrate particles in succession with individual layers by gas phase decomposition of volatile metal compounds in the presence of oxygen and/or water vapor or wet-chemically by hydrolytic decomposition of organic metal compounds.

8. The use of luster pigments as claimed in any of claims 1 to 6 for coloring paints, inks, including printing inks, plastics, glasses, ceramic products and decorative cosmetic preparations.

## Revendications

1. Pigments brillants à base de substrats métalliques sous forme de plaquettes revêtues plusieurs fois, qui présentent au moins un paquet de couches constitué
A) d'un revêtement incolore ayant un indice de réfraction n ≤ 1,8 et
B) d'un revêtement absorbant de façon sélective et ayant un indice de réfraction n ≥ 2,0, constitué essentiellement des oxydes absorbant de façon sélective que sont l'oxyde de fer(III), l'oxyde de chrome(III), l'oxyde de vanadium(V) et/ou l'oxyde de titane(III) ou des oxydes incolores que sont le dioxyde de titane et/ou l'oxyde de zirconium, qui ont été teintés à l'aide de colorants absorbant de façon sélective,
ainsi qu'éventuellement
C) d'un revêtement extérieur, incolore ou à absorption sélective, différent de la couche (B) située sous lui.

2. Pigments brillants selon la revendication 1, pour lesquels le revêtement (A) est une couche essentiellement constituée d'un oxyde métallique incolore ayant un faible indice de réfraction.

3. Pigments brillants selon la revendication 1 ou 2, pour lesquels le revêtement (A) est essentiellement constitué d'oxyde de silicium, d'oxyde de silicium hydraté, d'oxyde d'aluminium et/ou d'oxyde d'aluminium hydraté.

4. Pigments brillants selon l'une quelconque des revendications 1 à 3, pour lesquels le revêtement (C) est essentiellement constitué d'oxyde de silicium, d'oxyde de silicium hydraté, d'oxyde d'aluminium, d'oxyde d'aluminium hydraté, de dioxyde de titane, d'oxyde de zirconium, d'oxyde de fer(III) et/ou d'oxyde de chrome(III).

5. Pigments brillants selon l'une quelconque des revendications 1 à 4, qui ne contiennent qu'un paquet de couches formé de (A) et (B).

6. Pigments brillants selon l'une quelconque des revendications 1 à 5, pour lesquels le substrat métallique est essentiellement constitué de plaquettes en aluminium pouvant être passivé.

7. Procédé de préparation de pigments brillants selon l'une quelconque des revendications 1 à 6, caractérisé en ce que l'on recouvre les particules de substrat métallique avec des couches isolées, les unes après les autres, par décomposition en phase gazeuse de composés métalliques volatils, en présence d'oxygène et/ou de vapeur d'eau ou par voie chimique humide par décomposition par hydrolyse de composés métalliques organiques.

8. Utilisation de pigments brillants selon l'une quelconque des revendications 1 à 6, pour la coloration de laques, d'encres d'impression, d'encres, de matières plastiques, de verres, de produits céramiques et pour des préparations de cosmétique décorative.
